# EUROPEAN PATENT SPECIFICATION

(11) **EP 4 472 680 B1**
(45) Date of publication and mention of the grant of the patent: **27.08.2025**
(21) Application number: 23701521.9
(22) Date of filing: 24.01.2023
(51) Int. Cl.: A61L 2/00, A61L 2/10, A61L 2/24, A61L 9/20, F21V 33/00, H05B 47/115, H05B 47/12, H05B 47/125, H05B 47/13, H05B 47/175, F21V 21/088, F21V 23/04

(54) **UV-B LIGHT GENERATING SYSTEM**
UV-B LICHTERZEUGUNGSSYSTEM
SYSTÈME DE GÉNÉRATION DE LUMIÈRE UV-B

(30) Priority: 01.02.2022 EP 22154446
(43) Date of publication of application: 11.12.2024
(73) Proprietor: Signify Holding B.V., 5656 AE Eindhoven (NL)
(72) Inventor: WEIJERS, Aldegonda, Lucia, 5656 AE Eindhoven (NL)
(74) Representative: Verweij, Petronella Daniëlle
(86) International application number: PCT/EP2023/051631
(87) International publication number: WO 2023/148047

(56) References cited:
- WO-A1-01/02937
- WO-A1-2017/042662
- GB-A- 2 375 504
- US-A1- 2019 022 260
- US-A1- 2021 162 080
- US-A1- 2021 316 025
- US-A1- 2022 105 209

## Description

### FIELD OF THE INVENTION

The invention relates to a light generating system to provide ultraviolet light. The invention further relates to an arrangement comprising the light generating system. The invention further relates to a method for providing ultraviolet light.

### BACKGROUND OF THE INVENTION

Attachable devices are known in the art. US2017143868, for instance, describes an attachable and movable deodorizer for enclosed space comprising: a body having a placing space therein, a plurality of inlet holes arranged at the front thereof, and at least one outlet hole arranged aside; a fan disposed inside the placing space near the inlet holes; a photocatalyst filter disposed at the rear of the fan for the air sucked in by the fan to pass through the photocatalyst filter; an ultraviolet control circuit board including an ultraviolet-A LED lamp, a microcontroller, and a controlling chip; said ultraviolet control circuit board being disposed in the placing space and electrically connected to the fan for the ultraviolet-A LED lamp to illuminate and catalyze the photocatalyst filter for deodorization; a wire connected to the ultraviolet control circuit board with an end to supply electricity for the ultraviolet control circuit board and the fan; whereby when the body is disposed in an enclosed space of a trash bin or a shoes cabinet with a movable member for opening, the dusts and suspended substance filled within would be sucked into the body via the inlet holes by the fan, pass through the photocatalyst filter, and then be discharged from the body via the outlet hole; and the photocatalyst filter is able to purify the dirty air with stench chemical substance within the enclosed space.

WO 2017/042662 A1 discloses an ultrasound imaging system which includes a disinfection system. The disinfection system may include one or more ultraviolet light sources. The UV light sources may be included in a display. The disinfection system may be configured to operate when the display is parallel to a control panel of the ultrasound imaging system. The disinfection system may provide indications of the disinfection status of the ultrasound imaging system.

US2021316025 A1 discloses a germicidal system for use in disinfecting one or more contact surfaces and includes one or more germicidal devices each comprising a germicidal light source. The one or more germicidal devices may be connected to a network, which allows for controlling the operational parameters of the one or more germicidal devices and/or collecting information from the one or more germicidal devices.

US2021/162080 A1 discloses a portable light fastening assembly for use with the human interface of an electronic device and includes a lamp housing and an adjustable attachment device extending from the lamp housing. An ultra-violet (UV) light source is at least partially enclosed in the lamp housing such that the adjustable attachment device includes an engagement member and a receptacle housing such that the engagement member can be removably fastened within the receptacle housing for holding the lamp housing in a fixed position.

US2019/022260 A1 discloses a germicidal system for use in disinfecting a human interface device and includes at least one human interface device. One or more ultra-violet (UV) light sources are used in proximity to the at least one human interface device for disinfecting a touch surface of the human interface device below a surgical grade sterilization. A memory for storing usage data of the at least one UV light source. At least one server is used for providing a central storage location for usage data supplied from the memory and a computer is used in communication with the at least one server for controlling the operational parameters of the at least one UV light source.

### SUMMARY OF THE INVENTION

UV light has been used for disinfection for over 100 years. Wavelengths between about 190 nm and 300 nm may be strongly absorbed by nucleic acids, which may result in defects in an organism's genome. This may be desired for inactivating (killing), bacteria and viruses, but may also have undesired side effects for humans. Therefore, the selection of wavelength of radiation, intensity of radiation and duration of irradiation may be limited in environments where people may reside such as offices, public transport, cinema's, restaurants, shops, etc., thus limiting the disinfection capacity. Especially in such environments, additional measures of disinfection may be advantageous to prevent the spread of bacteria and viruses such as influenza or novel (corona) viruses like COVID-19, SARS, and MERS.

It appears desirable to produce systems, that provide alternative ways for air treatment, such as disinfection. Further, existing systems for disinfection may not easily be implemented in existing infrastructure, such as in existing buildings like offices, hospitality areas, etc. and/or may not easily be able to serve larger spaces. This may again increase the risk of contamination. Further, incorporation in HVAC systems may not lead to desirable effects and appears to be relatively complex. Further, existing systems may not be efficient, or may be relatively bulky, and may also not easily be incorporated in functional devices, such as e.g. luminaires.

Other disinfection systems may use one or more anti-microbial and/or antiviral means to disinfect a space or an object. Examples of such means may be chemical agents which may raise concerns. For instance, the chemical agents may also be harmful for people and pets.

In embodiments, the disinfecting light, may especially comprise ultraviolet (UV) radiation (and/or optionally violet radiation), i.e., the light may comprise a wavelength selected from the ultraviolet wavelength range (and/or optionally the violet wavelength range). However, other wavelengths are herein not excluded. The ultraviolet wavelength range is defined as light in a wavelength range from 100 to 380 nm and can be divided into different types of UV light / UV wavelength ranges (Table 1). Different UV wavelengths of radiation may have different properties and thus may have different compatibility with human presence and may have different effects when used for disinfection (Table 1).

**Table 1: Properties of different types of UV, violet, and NIR wavelength light**

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Near Infrared (deep red) | NIR | 780-950 | + | +/- | +++ | | |
| Violet | V | 380-420 | +/- | - | + | | |
| Ultra-violet A | UV-A | 320-380 | + | - | + | | |
| Ultra-violet B | UV-B | 280-320 | + | +/- | +/- | + | |
| Near ultraviolet C | Near UV-C | 230-280 | ++ | ++ | - | | |
| Far ultra-violet C | Far UV-C | 190-230 | +++ | +++ | + | | +/- |
| Extreme ultraviolet C | Extreme UV-C | 100-190 | +++ | +++ | - | | + |

Each UV type / wavelength range may have different benefits and/or drawbacks. Relevant aspects may be (relative) sterilization effectiveness, safety (regarding radiation), and ozone production (as result of its radiation). Depending on an application a specific type of UV light or a specific combination of UV light types may be selected and provides superior performance over other types of UV light. UV-A may be (relatively) safe and may inactivate (kill) bacteria, but may be less effective in inactivating (killing) viruses. UV-B may be (relatively) safe when a low dose (i.e. low exposure time and/or low intensity) is used, may inactivate (kill) bacteria, and may be moderately effective in inactivating (killing) viruses. UV-B may also have the additional benefit that it can be used effectively in the production of vitamin D in a skin of a person or animal. Near UV-C may be relatively unsafe, but may effectively inactivating, especially kill bacteria and viruses. Far UV-C may also be effective in inactivating (killing) bacteria and viruses, but may be (relatively to other UV-C wavelength ranges) (rather) safe. Far-UV light may generate some ozone which may be harmful for human beings and animals. Extreme UV-C may also be effective in inactivating (killing) bacteria and viruses, but may be relatively unsafe. Extreme UV-C may generate ozone which may be undesired when exposed to human beings or animals. In some application ozone may be desired and may contribute to disinfection, but then its shielding from humans and animals may be desired. Hence, in the table "+" for ozone production especially implies that ozone is produced which may be useful for disinfection applications, but may be harmful for humans / animals when they are exposed to it. Hence, in many applications this "+" may actually be undesired while in others, it may be desired. The types of light indicated in above table may in embodiments be used to sanitize air and/or surfaces.

The terms "inactivating" and "killing" with respect to a virus may herein especially refer to damaging the virus in such a way that the virus can no longer infect and/or reproduce in a host cell, i.e., the virus may be (essentially) harmless after inactivation or killing.

Hence, in embodiments, the light may comprise a wavelength in the UV-A range. According to the invention, the light comprises a wavelength in the UV-B range. In further embodiments, the light may comprise a wavelength in the Near UV-C range. In further embodiments, the light may comprise a wavelength in the Far UV-C range. In further embodiments, the light may comprise a wavelength in the extreme UV-C range. The Near UV-C, the Far UV-C and the extreme UV-C ranges may herein also collectively be referred to as the UV-C range. Hence, in embodiments, the light may comprise a wavelength in the UV-C range. In other embodiments, the light may comprise violet radiation.

Vitamin D deficiency can lead to a loss of bone density, which can lead to osteoporosis and fractures. Other symptoms may include muscle weakness, pain fatigue and depression. Further, vitamin D is known to have other advantages. Vitamin D is naturally produced in the skin on exposure to sunlight, or more specifically ultraviolet B radiation that the sun emits reacts with the protein 7-DHC in the skin, converting it to vitamin D3 (active form of vitamin D). UV-B radiation or UV-B radiation may be provided artificially to promote the generation of vitamin D in the skin. Further, it has been the standard therapy for skin diseases since its inception in the 1920s. Further, UV-B radiation may have other advantageous effect to the human body.

The urban work environment promotes an indoor lifestyle with relatively little exposure to natural light UV-B radiation. However, at the same time there are no provisions to provide this UV-B radiation in a safe and reliable manner, such as by means of securable device to the workplace such a cash register, a computer, a mirror, etc. Hence, there may be a desired to provide additional UV-B radiation. However, also in view of air treatment, UV radiation may desirable.

Devices that provide UV-B radiation may require a mechanism to turn off the provided UV-B radiation. Conventional mechanisms such as a switch may suffer from drawbacks. Typical mechanical switches may be expensive and complicated in construction. Further, a conventional mechanism lacks the ability to automatically detect and control delivery of UV-B radiation.

Hence, it is an aspect of the invention to provide UV-B radiation in a safe and reliable manner, which preferably further at least partly obviates one or more of above-described drawbacks. The present invention may have as object to overcome or ameliorate at least one of the disadvantages of the prior art, or to provide a useful alternative.

Hence, in a first aspect the invention is a light generating system according to claim 1.

In yet further embodiments, the lighting module may be functionally attachable to a display comprising device.

The light source may operate in an on-mode or an off-mode. In the former mode, the light source radiation is provided; in the latter mode the light source does not provide light source radiation. Hence, the light source may be configured to provide light in the on-mode and be switched off in the off-mode. According to the invention, the light source is configured to provide radiation with a wavelength in the range 280-320 nm. The term "operational mode" may especially refer to an on-mode.

The phrase "radiation having a wavelength in the 280-320 nm" may especially indicate that the light source is configured to generate light (or radiation) having at least intensity in the wavelength range of 280-320 nm. Hence, a spectral power distribution of the light source radiation may show intensity at one or more wavelengths in the wavelength range of 280-320 nm. Especially, the light source radiation may have a peak wavelength within the wavelength range of 280-320 nm. More especially, in embodiments more than 75%, such as more than 80%, even more especially at least 90% of the spectral power of the light source radiation may be within the wavelength range 280-320 nm.

The term "light source" may in principle relate to any light source known in the art. It may be a conventional (tungsten) light bulb, a low pressure mercury lamp, a high pressure mercury lamp, a fluorescent lamp, a LED (light emissive diode). In a specific embodiment, the light source comprises a solid state LED light source (such as a LED or laser diode (or "diode laser")). The term "light source" may also relate to a plurality of light sources, such as 2-200 (solid state) LED light sources. Hence, the term LED may also refer to a plurality of LEDs. Further, the term "light source" may in embodiments also refer to a so-called chips-on-board (COB) light source. The term "COB" especially refers to LED chips in the form of a semiconductor chip that is neither encased nor connected but directly mounted onto a substrate, such as a PCB. Hence, a plurality of light emitting semiconductor light source may be configured on the same substrate. In embodiments, a COB is a multi LED chip configured together as a single lighting module.

The light source may have a light escape surface. Referring to conventional light sources such as light bulbs or fluorescent lamps, it may be outer surface of the glass or quartz envelope. For LED's it may for instance be the LED die, or when a resin is applied to the LED die, the outer surface of the resin. In principle, it may also be the terminal end of a fiber. The term escape surface especially relates to that part of the light source, where the light actually leaves or escapes from the light source. The light source is configured to provide a beam of light. This beam of light (thus) escapes from the light exit surface of the light source.

The term "light source" may refer to a semiconductor light-emitting device, such as a light emitting diode (LEDs), a resonant cavity light emitting diode (RCLED), a vertical cavity laser diode (VCSELs), an edge emitting laser, etc. The term "light source" may also refer to an organic light-emitting diode (OLED), such as a passive-matrix (PMOLED) or an active-matrix (AMOLED). In a specific embodiment, the light source comprises a solid-state light source (such as a LED or laser diode). In an embodiment, the light source comprises a LED (light emitting diode). The terms "light source" or "solid state light source" may also refer to a superluminescent diode (SLED).

The term LED may also refer to a plurality of LEDs.

The term "light source" may also relate to a plurality of (essentially identical (or different)) light sources, such as 2-2000 solid state light sources. In embodiments, the light source may comprise one or more micro-optical elements (array of micro lenses) downstream of a single solid-state light source, such as a LED, or downstream of a plurality of solid-state light sources (i.e. e.g. shared by multiple LEDs). In embodiments, the light source may comprise a LED with on-chip optics. In embodiments, the light source comprises a pixelated single LEDs (with or without optics) (offering in embodiments on-chip beam steering).

In embodiments, the light source may be configured to provide primary radiation, which is used as such, such as e.g. a blue light source, like a blue LED, or a green light source, such as a green LED, and a red light source, such as a red LED. Such LEDs, which may not comprise a luminescent material ("phosphor") may be indicated as direct color LEDs.

In other embodiments, however, the light source may be configured to provide primary radiation and part of the primary radiation is converted into secondary radiation. Secondary radiation may be based on conversion by a luminescent material. The secondary radiation may therefore also be indicated as luminescent material radiation. The luminescent material may in embodiments be comprised by the light source, such as a LED with a luminescent material layer or dome comprising luminescent material. Such LEDs may be indicated as phosphor converted LEDs or PC LEDs (phosphor converted LEDs).

In other embodiments, the luminescent material may be configured at some distance ("remote") from the light source, such as a LED with a luminescent material layer not in physical contact with a die of the LED.

The light source may especially be configured to generate light source light having an optical axis (O), (a beam shape,) and a spectral power distribution. The light source light may in embodiments comprise one or more bands, having band widths as known for lasers.

The term "light source" may (thus) refer to a light generating element as such, like e.g. a solid state light source, or e.g. to a package of the light generating element, such as a solid state light source, and one or more of a luminescent material comprising element and (other) optics, like a lens, a collimator. A light converter element ("converter element" or "converter") may comprise a luminescent material comprising element. For instance, a solid state light source as such, like a blue LED, is a light source. A combination of a solid state light source (as light generating element) and a light converter element, such as a blue LED and a light converter element, optically coupled to the solid state light source, may also be a light source (but may also be indicated as light generating device). Hence, a white LED is a light source (but may e.g. also be indicated as (white) light generating device).

In embodiments, the light generating device may comprise a luminescent material. In embodiments, the light generating device may comprise a PC LED. In other embodiments, the light generating device may comprise a direct LED (i.e. no phosphor). In embodiments, the light generating device may comprise a laser device, like a laser diode. In embodiments, the light generating device may comprise a superluminescent diode. Hence, in specific embodiments, the light source may be selected from the group of laser diodes and superluminescent diodes. In other embodiments, the light source may comprise an LED.

The term "light source" herein may also refer to a light source comprising a solid state light source, such as an LED or a laser diode or a superluminescent diode.

The "term light source" may (thus) in embodiments also refer to a light source that is (also) based on conversion of light, such as a light source in combination with a luminescent converter material. Hence, the term "light source" may also refer to a combination of a LED with a luminescent material configured to convert at least part of the LED radiation, or to a combination of a (diode) laser with a luminescent material configured to convert at least part of the (diode) laser radiation.

In embodiments, the term "light source" may also refer to a combination of a light source, like a LED, and an optical filter, which may change the spectral power distribution of the light generated by the light source. Especially, the "term light generating device" may be used to address a light source and further (optical components), like an optical filter and/or a beam shaping element, etc.

The phrases "different light sources" or "a plurality of different light sources", and similar phrases, may in embodiments refer to a plurality of solid-state light sources selected from at least two different bins. Likewise, the phrases "identical light sources" or "a plurality of same light sources", and similar phrases, may in embodiments refer to a plurality of solid-state light sources selected from the same bin.

The term "solid state light source", or "solid state material light source", and similar terms, may especially refer to semiconductor light sources, such as a light emitting diode (LED), a diode laser, or a superluminescent diode. Especially, the term "light source" herein may refer to a light emitting diode (LED), a diode laser, or a superluminescent diode. For instance, the light source may comprise one or more LEDs.

According to the invention, the light generating system further comprises a proximity sensor. In embodiments, the proximity sensor may be configured to detect the presence of a (nearby) entity (in the field of view of the proximity sensor). Especially, an entity may herein refer to an object, a human, or an animal. Especially, the proximity sensor may at least be configured to detect the presence of a human in the field of view of the proximity sensor. The term "entity" may also refer to a plurality of entities.

According to the invention, the proximity sensor is configured to generate a related signal when detecting an entity (in the field of view of the proximity sensor). In embodiments, the proximity sensor may be configured to provide a signal based on the proximity of entities to the proximity sensor. In embodiments, the proximity sensor may be configured to distinguish between entities in its vicinity such as between objects and humans.

In embodiments, an identification of a human and/or an animal in close proximity to the proximity sensor may be useful to ensure that radiation is provided based only on the proximity or non-proximity of the human and/or an animal, such as a user, to the proximity sensor and not based on other objects in the field of view of the proximity sensor. Here "user" may especially refer to a person using the light generating system and/or to a person using the display comprising device.

According to the invention, the proximity sensor is configured to detect the presence of objects (to the exclusion of living entities) in the vicinity of the proximity sensor. The detection of an entity, such as an object, in the vicinity of the proximity sensor may be advantageous in a situation where the light source is completely blocked by an entity, such as an object, in close proximity to the proximity sensor, in which case, the light source may be configured to be operated in the off-mode.

In embodiments, the lighting module may comprise a combination of proximity sensors to detect objects and persons. This may be advantageous, where one proximity sensor may provide a related signal based on the proximity of a person to the proximity sensor, and another proximity sensor may provide a related signal based on the proximity of objects in the vicinity of the proximity sensor.

In embodiments, the proximity sensor may be selected from a group comprising a camera, a passive infrared sensor, an ultrasonic sensor, a microwave sensor, a time of flight sensor, and an audio sensor.

In embodiments, the proximity sensor may be a camera such as especially a digital camera or a LiDAR. In yet further embodiments, the camera may be configured to provide a related proximity sensor signal to the control system. The proximity sensor signal in this case may be image data or a continuous stream of image data.

In further embodiments, the proximity sensor may be a passive infrared sensor. A passive infrared sensor may be configured to measure infrared light radiating from objects or users in its field of view. Changes detected by the passive infrared sensor may depend on the temperature and the surface characteristics of the objects or users in the field of view of the proximity sensor. These changes may trigger a related proximity sensor signal, that may be provided to the control system.

An ultrasonic sensor may be a sensor configured to detect distance to objects using ultrasonic waves. Ultrasonic waves are sound waves that may in embodiments have a frequency of 18kHz or higher. The ultrasonic sensor may convert electrical energy to sound, emit the sound waves in a targeted direction, and convert the echo back to electrical signal to determine the distance to an obstacle. Hence, an ultrasonic sensor may be used to detect the presence of an object or person in the vicinity of the sensor and provide a related proximity sensor signal.

An audio sensor may sense e.g. the use of a keyboard, breathing, talking, movements. In dependence thereof, the control system may control the UV radiation.

In embodiments, a microwave sensor may be configured to detect heat. These sensors may emit a series of directional beams that detect the temperature of an incident object. A change in the temperature detected, triggers the sensor to provide a related proximity sensor signal to the control system.

A time of flight sensor may be used to determine depth information. The time of flight sensor may be configured to emit a light signal, specifically an infrared light signal in the field of view of the time of flight sensor. The time taken for the infrared signal to bounce back is recorded. This phase shift in the reflected infrared light may be used to infer distance from an entity. The time of flight sensor may provide a related proximity sensor signal on detecting an object or person in close proximity to the sensor.

A time of flight (TOF) sensor may comprise in embodiments a source of IR radiation, such as an IR LED. However, the TOF sensor may also be based on other wavelengths than IR. In embodiments, the proximity sensor may especially comprise an IR TOF sensor.

Hence, the term "proximity sensor" may essentially refer to any sensor from which the proximity of an entity may be determined. The proximity sensor according to the invention, estimates a distance of the entity to the proximity. In embodiments, the proximity sensor may generate a signal when there is a change, such as in the case of a movement sensor, or such as may be the case with sensors based on temperature.

In yet further embodiments, the control system may process three dimensional time resolved positional information such as from the LiDAR to uniquely determine the position and orientation of different entities over time. In yet further embodiments, the control system may interpret proximity sensor signal from a proximity sensor such as a digital camera and use an image processing algorithm to identify positional information of entities in the field of view of the proximity sensor. In further embodiments, the proximity sensor may be calibrated on a first use, where the user may position themselves at a predetermined distance from the proximity sensor, such as a camera. On subsequent usage, the distance of the user from the proximity sensor may be determined based on the area of the field of view occupied by the user.

The light source may provide a beam of UV radiation. The beam may be defined by a full width half maximum (FWHM). In embodiments, the full width half maximum defined beam of UV radiation may substantially overlap with the field of view of the proximity sensor. Or, in embodiments, the field of view of the proximity sensor may substantially overlap with the full width half maximum defined beam of UV radiation. In embodiments, within the predetermined, in a cross-sectional view perpendicular to an optical axis of the beam of UV radiation, at least 50%, such as at least about 65%, of the cross-section of the beam of UV radiation may overlap with the cross-section of the field of view, or at least 50%, such as at least about 65%, of the cross-section of the field of view may overlap with the beam of UV radiation. Here, the term "field of view" may especially refer to the solid angle within which a sensor may receive an input.

Especially, the optical axis may be defined as an imaginary line that defines the path along which light propagates through a system starting from the light generating element, here especially the light source. Especially, the optical axis may coincide with the direction of the light with the highest radiant flux.

The term "proximity sensor" may also refer to a plurality of (different) proximity sensors. Using a combination of two or more proximity sensors, especially two or more different types of proximity sensors, may be advantageous in providing light source radiation to the user who may spend time in front of the light generating system as opposed to other objects in the field of view of the proximity sensor, such as for example, using only an infrared proximity sensor may be unable to distinguish between the user and a relatively hot coffee mug placed before the light generating system.

According to the invention, the light generating system further comprises the control system. In embodiments, the control system may be functionally coupled to the proximity sensor and the light source. In embodiments, the control system may be configured to receive a proximity sensor signal from the proximity sensor. The control system may be functionally coupled to the proximity sensor via an electrically conductive connection or via an optical connection, though other connections, like via (other) waves, like Wi-Fi or Bluetooth, may also be possible.

The term "controlling", and similar terms, may especially refer at least to determining the behavior or supervising the running of an element. Hence, herein the term "controlling", and similar terms, may e.g. refer to imposing behavior to the element (determining the behavior or supervising the running of an element), etc., such as e.g. measuring, displaying, actuating, opening, shifting, changing temperature, etc. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions from a remote control. In embodiments, the control system may be controlled via an App on a device, such as a portable device, like a Smartphone or I-phone, a tablet, etc.. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, Wifi, ZigBee, BLE or WiMax, or another wireless technology.

The system, or apparatus, or device may execute an action in a "mode" or "operation mode" or "operational mode" or "mode of operation" or "control mode". Likewise, in a method an action or stage, or step may be executed in a "mode" or operation mode" or "operational mode" or "mode of operation" or "control mode". The term "mode" may also be indicated as "controlling mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

Hence, in embodiments, the control system may control in dependence of one or more of an input signal of a user interface, a sensor signal (of a sensor), and a timer. The term "timer" may refer to a clock and/or a predetermined time scheme.

In embodiments, the light source may be configured to be in the on-mode or off-mode simply based on the related proximity sensor signal without further processing of the signal. Hence, in specific embodiments the control system may impose either the on-state or the off-state on the basis of the proximity sensor signal. According to the invention, however, the control system also controls the intensity of the light source in dependence of the proximity sensor signal (see further also below).

In further embodiments, the control system may be configured to process the proximity sensor signal. This may enable the control system to distinguish between entities, such as objects and humans. Further, the control system may use the proximity sensor signal to determine the distance of the user from the proximity sensor and the duration for which the user has been exposed to the light source. In embodiments, the control system may regulate the operation of the light source based on information obtained from processing the proximity sensor signal, such as to vary the intensity of the light source and the duration for which light source radiation is provided.

In embodiments, the control system may be configured to derive positional information of the entities in the field of view of the proximity sensor based on the proximity sensor signal.

The term "positional information" may relate to one or more of distance of an entity from the proximity sensor, the solid angle covered by an entity, a translational motion, and a rotational motion of an entity in the field of view of the proximity sensor. The control system may operate in different levels of fidelity based on the type of proximity signal received.

In embodiments, the control system may configure the light source based on the proximity of entities in the vicinity of the proximity sensor without distinguishing between different types of entities. In further embodiments, the control system may configure the light source based on a combination of two or more types of proximity sensor signals for example, a combination of a time of flight sensor and an infrared sensor may help uniquely distinguish a human from other objects in the field of view of the proximity sensor.

In embodiments, the control system may use the proximity sensor signal from a multitude of (different types of) proximity sensors to identify positional information over time of different entities in the field of view of the proximity sensor.

In embodiments, the control system may comprise a local control system and a remote control system. In further embodiments, the remote control system may be in communication with the local control system. In yet further embodiments, the remote control system may be configured to switch between the on-mode and off-mode of the light source. In yet further embodiments, the remote control system may be used to regulate the intensity of light source radiation, or the duration for which the light source radiation is provided, or a combination thereof. In embodiments, the remote control system may communicate with the local control system wirelessly by means of wireless technology. In embodiments, the wireless communication may occur over a Wi-Fi network. Wi-Fi is a family of wireless network protocols, based on the IEEE 802.11 family of standards, which facilitates local area networking, allowing communication system to exchange data by radio waves. In further embodiments, the remote control system may use broadband cellular technology such as 4G or 5G which allow transport of multiple signals at a wide range of frequencies and enables multiple messages to be sent simultaneously. In yet further embodiments, the remote control system may use Bluetooth, which is a short-range wireless technology standard that is useful for exchanging data over short distances using high frequency radio waves from 2.402 GHz to 2.48 GHz. Wireless communication may provide the advantage of altering the mode of operation or the system, for example to switch between the on and off-mode of the light source, and adjusting one or more parameters of the light source or the proximity sensor remotely. In further embodiments, the remote control system may communicate with the local control system by means of wired communication such as over ethernet or USB.

In embodiments, the light generating system may comprise an electrical power source. Especially, in embodiments the lighting module may comprise the electrical power source. In embodiments, the electrical power source may be electrically connected to the light source, or the proximity sensor, or a combination thereof. In embodiments, the electrical power source may be configured to provide electrical power to the light source, the control system and the proximity sensor. In embodiments, the power source may be a portable power source. The electrical power source may be rechargeable. In embodiments, the lighting module may be charged wirelessly using charging pads that use tightly-coupled electromagnetic inductive or non-radiative charging. In further embodiments, charging bowls or through-surface type chargers may be used that facilitate loosely-coupled or radiative electromagnetic resonant charging, especially for charging up to a few centimeters away. In yet further embodiments, uncoupled radio frequency wireless charging may be used that allows charging at distances of many feet. In other embodiments, the power supply may be wired, where the power supply unit draws power from an external source by means of wires that can be plugged into an external power source. In yet further embodiments, the electrical power source may draw power from a combination of wired and wireless sources.

According to the invention, .the lighting module is functionally attachable to a display comprising device. In embodiments, the lighting module may be functionally coupled to a display comprising device.

However, the lighting module may also be attached to other surfaces or objects. For instance, the user may spend a significant amount of time, such as at a cash register (such as in a shop), a piano, a mirror, light fixtures, etc.

In embodiments, the light generating system may further comprise attaching elements selected from the group of a rail, a magnet, a clamping element, a snapper element, and Velcro. In further embodiments, the attaching elements may be functionally coupled to the lighting module.

In embodiments, the light generating system may comprise a rail. In further embodiments, the lighting module may have a set of rails attached to the back end of the lighting module. In embodiments, the display comprising device may have analogous rails. In embodiments, the rails attached to the lighting module may be used to slide and secure the light generating system to the display comprising device. In further embodiments, the light generating system may comprise a magnet as an attaching element. The magnet may provide the advantage of easily securing as well as removing the light generating system from the display comprising device. This provides the advantage that the display comprising device such as a laptop, wherein the light generating system may have to be removed from the laptop before the laptop may be closed. However, the use of magnetic attaching elements may affect the display of the display comprising device, in which case it may be advantageous to use other attaching elements. The magnetic attaching elements may be used when attaching the lighting module to a non-display comprising device, such as a mirror, a cash register, a piano, etc. In yet further embodiments, the attaching element may be a clamping element or a snapper element. Certain display comprising devices may not possess analogous constructions to secure the lighting module to them. These elements provide the advantage of possessing clamps or snappers which may be used to secure the lighting module to the display comprising device, without the need for attaching additional functional elements to the display comprising device. In yet further embodiments, the attaching element may be Velcro. In yet further embodiments, the male part of the Velcro may be attached onto the lighting module with the female part of the Velcro attached to the display comprising device. The Velcro may further allow for multiple positions in which the light generating system may be secured. This may also allow the light generating system to be held in a specific orientation. This is useful for specific situations where higher fidelity is required in the positioning of the lighting module. Other male-female connections may also be possible.

In embodiments, one or more of the proximity sensor and the control system may be configured external from the lighting module. In embodiments, the display comprising device may comprise the control system. In other embodiments, the display comprising device may comprise the proximity sensor. In further embodiments, the display comprising device may comprise a both the control system and the proximity sensor. For example, a device such as a smart phone, or a computer (or a display functionally coupled to a desktop computer), or laptop, or tablet may comprise the control system. Further, these devices may comprise a camera which may be used as a proximity sensor. Hence, in specific embodiments the proximity sensor may be provided by a camera of the display comprising device. However, in other embodiments the proximity is not the camera of the display comprising device to which it may be functionally coupled. Hence, an arrangement comprising the display comprising device and the module may comprise a camera provided by the display and a proximity sensor comprised by the module, which proximity sensor may (also) comprise a camera or may not comprise a camera.

The lighting module may be in wired or wireless communication with the control system in these devices. In embodiments, the control system may comprise an application or program to control the operation of the light source. This may provide the advantage of reducing the complexity of construction and having a more compact lighting module. Further, these devices may provide the advantage of processing higher fidelity proximity sensor signals, while ensuring no increase in complexity of the lighting module.

In embodiments, the lighting module may comprise the proximity sensor. In other embodiments, the lighting module may comprise the control system.

In embodiments, the lighting module may comprise a housing at least partially enclosing the light source. In specific embodiments, the housing may also at least partially enclose the proximity sensor. Yet, in specific embodiments the housing may at least partially, such as essentially fully, enclose the control system. Further, the housing may in embodiments at least partly enclose communication element, for communication with devices external of the lighting module, like in embodiments a computer.

In embodiments, the light generating system may be a stand-alone unit comprising the proximity sensor and the control system, where the lighting module may comprise the proximity sensor and the control system. This may provide the advantage of providing a light module that may function independent of other devices. This may also improve the versatility in the different locations in which the light generating system may be used, such as for example the lighting module attached to a reading lamp.

In embodiments, the lighting module may be configured to generate in a first operational mode the UV radiation and in a second operational mode visible light.

In further embodiments, the control system may be configured to operate the lighting module in the first operational mode, or the second operational mode, or both operational modes. In further embodiments, the remote control system may be used to select the operational mode i.e. the first operational mode, or the second operational mode, or a combination thereof.

In embodiments, the visible light may be white light having a color rendering index (CRI) of at least 75, such as at least about 80. In specific embodiments, the CRI may be at least 85. In embodiments, the correlated color temperature may be selected from the range of 2000-6500 K, especially selected from the range of 2700-6500 K.

In embodiments, the light source may be configured to provide visible light having a wavelength in the range 380-780 nm.

In embodiments, the first operational mode and the second operational mode may be executed during non-overlapping time periods, and in other embodiments the first operational mode and the second operational mode may be executed during overlapping time periods. In further embodiments, the lighting module may switch between a first operational mode and a second operational mode.

In embodiments, operating only in the second operational mode may provide the advantage of providing visible light without providing ultraviolet light, and vice versa. In yet further embodiments, the lighting module may be operated in both the first operational mode and the second operational mode. This may provide the advantage of providing UVB radiation in addition to visible light, in situations such as operating the lighting module at night or in low light conditions. In embodiments, the lighting module may be configured to shine visible light on the user, especially white light (see also above). This may provide the advantage of illuminating the face of the user such as for visibility during a video call. Further, this may also be used in its capacity as a flashlight or a reading light. In further embodiments, the UV radiation may also be used for sterilization. This may be useful for sterilizing a keyboard in front of a display.

In further embodiments, the light source radiation may be aligned downwards to avoid shining light directly into the eyes of the user. The display comprising device may be adjustable to a suitable viewing angle, thus, lighting module attached to the display comprising device, may not be aligned optimally to provide UV-B radiation to the user. This may inadvertently provide UV-B radiation, such as onto the eyes of the user. Hence, in embodiments, the lighting module may comprise one or more of a mirror, or a prism, or a combination thereof. The mirror or the prism may promote safe and reliable use of the device by directing the emitted light away from the eyes of the user. In further embodiments, the mirror or the prism may be used to direct light onto a particular region in front of the light source. In specific embodiments, such optical elements may be controlled in dependence of the proximity sensor and/or in dependence of one or more other sensors. Alternatively or additionally, the module may have an adjustable position relative to the display comprising device, such as via an adjustable hinge. In more specific embodiments, the position of the module relative to the display comprising device may be controlled in dependence of the proximity sensor and/or in dependence of one or more other sensors.

In embodiments, the light generating system comprises a second light source configured to generate the visible light. In more specific embodiments, the second light source is comprised by the lighting module. In relation to light sources, it is especially refer to the above passage with embodiments about light sources. Especially, the second light source (also) comprises a solid-state light source, such as a laser, a LED, or a superluminescent diode. As can be derived from the above, the term "second light source" may also refer to a plurality of second light sources.

In general, the (first) light source and the second light source are different light sources. Especially, in embodiments the first light source and the second light source may be controlled individually. However, in other specific embodiments the first light source may be configured to generate the UV radiation and the visible light. For instance, a single light source may provide the UV radiation and the visible light. Especially, however, the first light source and the second light source are different light sources, which may be controlled individually.

In embodiments the light generating system may further comprise a slider element ("slider"). For instance, a slider element may be useful to block the first light source and/or the proximity sensor. Blocking the former may be desirable in view of safety and/or blocking the latter may be desirable in view of privacy. Further, it may be desirable to combine an off-function and/or an on-function with the position of the slider element.

Especially, the slider element may be slidable in a plane, with a single degree of freedom. Hence, the slider element may be translated from one position to another position via a translation. A position may block the proximity sensor and optionally the light source, another position may block the light source and optionally the proximity sensor, and yet another position may not block the light source nor the proximity sensor.

Especially, in embodiments the slider element may be slidable between (i) a first position wherein the slider element may neither be configured downstream of the proximity sensor nor of the light source and (ii) a second position wherein the slider element is configured downstream of the proximity sensor. Further, in the second position, the control system may be configured to keep the light source in the off-mode in dependence of the proximity sensor signal.

Hence, in embodiments the signal generated by the proximity sensor when the slider element is in the second position may trigger the control system to switch off or keep switched off the light source. Further, in embodiments the signal generated by the proximity sensor when the slider element is in the first position may trigger the control system to switch on or keep switched the light source on.

Hence, in embodiments the operation of the lighting module may also be controlled by means of the slider element. Especially, the slider element may comprise a part to block the field of view of the proximity sensor and the light source. The slider element may thus be configured in a number of positions such as at least two positions. In embodiments, the positions of the slider element may be discrete i.e. they lock into specific positions. In other embodiments, the position of the slider may be a plurality of positions. Further, the positions may be interpreted as a region of extension of the slider element. Hence, in embodiments the term "position" may refer to a plurality of positions.

In the first position, the slider element may not obstruct field of view of the proximity sensor and the light source. In the second position, the slider element may be configured downstream of only the proximity sensor. The proximity sensor may detect the presence of an object when its field of view is obstructed by the slider element. In this configuration, the proximity sensor may generate the related proximity sensor signal to indicate the presence of an object in its close vicinity. The light source may be (configured to be) in the off-mode based on this related proximity sensor signal. This may provide the user the advantage of turning the light source off without the requirement of removing the lighting module. Further, this may provide the advantage of allowing the user to be in the vicinity of the light generating system by providing a manual mechanism to configure the light source to be in the off-mode. Further, the slider element may help protect the components of the light generating system when not in use. The slider element may also increase the lifetime of the light source as the light source may only operate when needed.

In embodiments, the slider element may be slidable between (i) the first position and (iii) a third position, wherein (in third position) the slider element may be configured downstream of both the proximity sensor and the light source. In embodiments, the slider element may be configurable in two positions, the first position and the third position. This may provide the advantage of (manually) blocking the light source when not required. This may further improve the safety and reliability of the light generating system, by providing the user further manual control over the light source such as, manually obstructing the light source. This may also be advantageous as a failsafe to prevent injury from exposure to ultraviolet light in the event of failure of the one or more elements in the light generating system.

In embodiments, the slider element may be configured manually i.e. the user may physically slide the slider element to one or more positions. In embodiments, the slider element may be configured electronically. In further embodiments, the light generating system may comprise a motor, such as a stepper motor, which may be functionally coupled to the slider element. In yet further embodiments, the control system may configure the motor to slide the slider element to one or more positions.

Hence, in embodiments the signal generated by the proximity sensor when the slider element is in the third position may trigger the control system to switch off or keep switched off the light source. Further, in embodiments the signal generated by the proximity sensor when the slider element is in the first position may trigger the control system to switch on or keep switched the light source on (see also above).

In embodiments, the slider element may be slidable in a fourth position downstream of the light source and in one or more other positions. In further embodiments, the light generating system may further comprise a tactile switch, wherein the slider element may be configured to contact the tactile switch when positioned in the fourth position. In yet further embodiments, upon contacting the tactile switch, the control system may be configured to keep or switch the light source to the off-mode.

In embodiments, the fourth position may be a position of the slider element configured in between the proximity sensor and the light source. However, alternatively or additionally the slider element may be configured in a fourth position downstream of both the proximity sensor and the light source (i.e. especially the third position as described above). In yet other embodiments, the fourth position may be a position downstream of at least the proximity sensor (i.e. essentially the second position as described above). In yet other embodiments, the fourth position may be a position downstream of the light source only. In yet other embodiments, the fourth position may be a position downstream of the light source of the lighting module without a proximity sensor (or even a light generating system without a proximity sensor).

The tactile switch may be an element that when physically contacted may complete an electronic circuit or provide a related tactile switch signal to the control system. The control system in embodiments, may be configured to operate the light source in the off-mode based on the related tactile switch signal. This may allow the user to manually generate the related tactile switch signal to configure the light source in the off-mode. In embodiments, the tactile switch may be coupled directly to the light source and not the control system. This may provide the user further control over the system. Further, this may act as a failsafe in the event that the related proximity sensor signal is not generated. This may also be advantageous in the event that the control system fails to configure the light source to the off-mode based on the related proximity sensor signal. Yet further, the fourth position may be the end position of the slider element, which may improve the ease of use of the light generating system. Being the end position, the user may not be required to check the particular position of the slider element while turning the light generating system to the off-mode. Rather, the user may simply slide the slider element to its extreme position. The term "end position" may refer to a position of the slider element wherein the slider element is fully closed.

As also indicated above, in the first position of the slider element, the proximity sensor and the light source may not be obstructed and the device may provide UV light. In the second position, the slider element may obstruct the proximity sensor but not the light source and hence, the light generating system may be configured to be in the off-mode. In the third position, the slider element may obstruct both the proximity sensor and the light source, which may configure the system to be in the off-mode. In the fourth position, the slider element may obstruct the light source, and further contact the tactile switch. In this position, the light generating system may be configured to operate in the off-mode.

In yet another embodiment, the light generating system may further comprise a tactile switch, wherein the slider element is configured to contact the tactile switch when positioned in the first position (i.e. neither downstream of the sensor, nor downstream of the light source. Especially, upon contacting the tactile switch, the control system may be configured to keep or switch the light source in the on-mode only when the slider element is configured in the first position. This may be another way to provide (additional) safety and/or user control to the system.

In yet another embodiment, the light generating system may further comprise a tactile switch, wherein the slider element is configured not to contact the tactile switch when positioned in the first position, but to contact the tactile switch in any other position. Especially, upon contacting the tactile switch, the control system may be configured to keep or switch the light source in the on-mode only when the slider element is configured in the first position. This may be another way to provide (additional) safety and/or user control to the system.

In again yet other embodiments, the slider element may be slidable in a fourth position downstream of the light source and in one or more other positions, wherein the light generating system further comprises a tactile switch, wherein the slider element is configured to contact the tactile switch when positioned only in the fourth position; wherein upon contacting the tactile switch, the control system is configured to keep or switch the light source in the off-mode. Hence, when the slider is not configured downstream off the light source, the control system may keep or switch the light source in the on-mode, but only when configured downstream of the light source, the light source will be switch off.

In again yet other embodiments, the slider element may be slidable in a fourth position downstream of the light source and in one or more other positions, wherein the light generating system further comprises a tactile switch, wherein the slider element is configured to contact the tactile switch when positioned in any other position than the fourth position; wherein upon not contacting the tactile switch, the control system is configured to keep or switch the light source in the off-mode. Hence, when the slider is not configured downstream off the light source, the control system may keep or switch the light source in the on-mode, but only when configured downstream of the light source, the light source will be switch off.

Especially, the first position and the second position are not overlapping positions. Further, when the first position includes a plurality of first positions and/or the second position includes a plurality of second positions, especially the first position and the second position are not overlapping positions.

Especially, the first position and the third position are not overlapping positions. Further, when the first position includes a plurality of first positions and/or the third position includes a plurality of third positions, especially the first position and the third position are not overlapping positions.

Dependent upon the configuration of the lighting module, the second position and the third position may be different or may overlap. Further, when the second position includes a plurality of second positions and/or the third position includes a plurality of third positions, in embodiments one or more second positions may overlap with one or more third positions, and in other embodiments none of the one or more second positions and the one or more third positions may overlap.

The fourth position is herein especially introduced to indicate a position different from other positions. Hence, dependent upon the embodiments, the fourth position could be a first position, a second position, a third position or another position.

In embodiments, the light source, at maximum power (of the light source), may be configured to provide the UV radiation with a maximum radiant exposure H_{a,max} within a predetermined range (dₚ). In further embodiments, the maximum radiant exposure E_{a,max} may be selected from the range of up to 30 mWₐ/m², and the predetermined range (dₚ) may be selected from the range of up to 100 cm (i.e. 0-100 cm, more especially larger than 0 cm and up to 100 cm). In embodiments, the predetermined range (dₚ) may be selected from the values of 20 cm or 100 cm. In other embodiments, the predetermined range (dₚ) may be selected from the range of 50-100 cm. Even values up to about 150 cm may be chosen, e.g. for safety reasons.

UV-B radiation has been identified to be relatively safe and has been a part of treatment of skin diseases for many decades. However, exceeding a recommended dosage may cause injury. Hence, in embodiments, the light generating system may be configured to provide a maximum UV irradiance E_{a,max}. Exposure to radiation is dependent on the surface area of exposure. Hence, the amount of exposure to UV radiation may be indirectly dependent on the distance of the user from the light source. Hence, in embodiments the light generating system may be configured to provide a maximum UV irradiance E_{a,max} within a predetermined range (dₚ). The intensity of the UV radiation provided may be selected in the range up to 30 mWa/m², such as 1-30 mWa/m², and the intensity may be varied based on the predetermined distance (dₚ) of up to 10 cm, such as about 20 cm or about 100 cm. Hence, in embodiments the light source may be configured to provide a maximum UV irradiance E_{a,max} of at maximum 30 mWa/m², when measured at a distance selected from the range of 20-100 cm. This may provide the advantage of preventing injury by over exposure to UV radiation. The exposure H_{a,max} and predetermined distance may be a setting of the light source.

Irradiance is in the art also indicated as Eₑ, and may be defined as the radiant flux received by a surface per unit area. The indication "a" in e.g. Wa may be used to indicate the "actinic" radiation.

According to the invention, the proximity sensor is configured to determine a distance (dₒ) between the object and the light source. According to the invention, control system is configured to determine a dosage Dₐ of UV radiation received by the object on the basis of (a) a radiant flux of the UV radiation over time and (b) the sensor signal of the proximity sensor. According to the invention, the control system is configured to relate the dosage Dₐ of UV radiation received by the object to a predetermined safe dosage limit Dₛ and on the basis thereon control the radiant flux of the UV radiation.

According to the invention, the proximity sensor is configured to determine a distance (dₒ) to an object, more specifically the user. The proximity sensor may be physically very close to the light source. Hence, the distance (dₒ) may be a proxy for the distance between the light source and the user. The control system, according to the invention, is configured to determine a dosage Dₐ of UV radiation received by the object. The control system may infer the distance of the object over time from the proximity signal. This in combination with the radiant flux of the UV radiation provided may be used by the control system to determine the dosage Dₐ of UV radiation received by the object, especially the user. The control system further regulates the radiant flux of the UV radiation provided to ensure that the dosage Dₐ provided does not exceed a threshold Dₛ of UV radiation, where Dₛ may be a predetermined safe dosage. This may provide the advantage of ensuring that the light generating system functions within safe limits, so as to not injure the user by over exposure to UV radiation.

According to the invention, the proximity sensor is configured to estimate an average distance over time d_{o,a} between the object and the light source. According to the invention, the control system is configured to estimate a dosage Dₐ of UV radiation received and to be received by the object on the basis of (i) a radiant flux of the UV radiation over time and (ii) the sensor signal of the proximity sensor. According to the invention, the control system is configured to relate the dosage Dₐ of UV radiation received by the object to a predetermined safe dosage limit Dₛ and on the basis thereon control the radiant flux of the UV radiation.

The user using the lighting generating system for a certain duration of time may have already received a certain dosage of UV radiation. In embodiments, the control system may be able to estimate this dosage based on distance of the user to the light source and the duration of exposure. This information may be obtained based on a related proximity sensor signal. The control system, in embodiments, may be able to (further) estimate based on the predetermined safe dosage limit (Dₛ) the amount of UV radiation that may be further provided. Based on this information, the control system may regulate the amount of UV radiation that may be further provided. In addition, the control system may also configure the duration for which the UV radiation may be further provided. In embodiments, the control system may perform a feed forward operation, where future exposure to radiation may be regulated based on the amount of UV radiation already provided. Upon providing UV radiation such that the dosage of UV radiation provided is close to the safe dosage limit Aₛ, the control system may configure the light source to gradually reduce the intensity of UV radiation provided, until eventually when the light source may be switched to the off-mode. Thus, preemptively protecting the user from unsafe exposure to UV radiation.

In embodiments, the control system may be configured to switch the light source to the off-mode based on detecting the object within a predetermined minimum distance d_{o,min} from the light source during a predetermined minimum duration t_{o,min}. Especially, the predetermined minimum distance d_{o,min} is selected from the range of 0-25 cm, such as 0-20 cm, and wherein the predetermined minimum duration t_{o,min} is at least 0.1 seconds, more especially at least 0.5 seconds. Hence, when the object is observed for longer than e.g. 0.1 second within a distance of 20 cm from the proximity sensor, the control system may switch of the light source.

In embodiments, based on positional information from the related proximity sensor signal, the control system may estimate the distance of an entity from the proximity sensor. In embodiments, the control system may be configured to turn the light source to the off-mode on detecting an object within a distance d_{o,min}. In embodiments, the minimum distance may be selected from the range of 0-20 cm, such as 0-15 cm. The dosage of the UV-B radiation may be higher at close distances. A higher than recommended dosage may lead to injury. Thus, turning the light source to the off-mode may improve the safety of the system. In further embodiments, the control system may be configured to turn the light source to the off-mode based on a minimum exposure duration t_{o,min}. In embodiments, based on a related proximity sensor signal, the control system may infer the duration for which an obstacle resides within the field of view of the proximity sensor. In embodiments, the control system may be configured to turn the light to the off-mode on detecting the presence of an obstacle for a duration longer than t_{o,min}. This may provide the advantage of filtering between quick accidental movements of the user and prolonged exposure to the light source. On using the lighting module, the user may be placed in close proximity to the light source. In embodiments, the lighting module may detect the positional information of the user and deliver a recommended dosage of UV-B radiation. Further, the lighting module may be able to distinguish between quick movements such as motion of limbs or appendages in the close vicinity of the light source and prolonged exposure, such as when the user moves closer to the light source. In yet further embodiments, the control system may be configured to turn the light to the off-mode on detecting the presence of a user or person within a distance d_{o,min}, for a duration of time longer than t_{o,min}. This may provide the advantage of providing a uninterrupted stream of UV-B radiation to the user i.e. the operational mode of the light source is only changed when the user moves closer or further away from the proximity sensor as opposed other movements (such as limbs or appendages).

In another aspect, the invention may provide an arrangement comprising the light generating system of claim 1 and a display comprising device. According to this aspect, the lighting module is attached to the display comprising device.

In embodiments, the display comprising device may be selected from the group of a self-standing monitor, a supported monitor or display, a laptop, and a tablet. In further embodiments, the display comprising device may be a mobile phone, such as a smart phone. In yet further embodiments the display comprising device may also be a wall or ceiling mounted monitor. According to an aspect of the invention, the lighting module is attached to the display comprising device. A supported monitor or display may be a monitor or display attached to a wall or other support element, like a ceiling, a building support element, a wall of an elevator, etc.

Here, "smart phone" refers to a cellular phone with an operating system capable of running an application, such as an I-phone, where the mode of interaction with the device is by means of a display. Attaching the lighting module to the display comprising device may be advantageous since the user may already spend several hours in front of the said display comprising device(s) as a part of their daily routine.

In embodiments, the lighting module may be functionally attached to a display comprising device. The lighting module may be configured to provide ultraviolet light to the user while they operate a device comprising a screen. The user of these devices may spend a significant amount of time in close proximity to a screen. This provides the advantage of safely providing UV-B radiation as a part of the user's daily routine.

In embodiments, the lighting module may be secured fast to the display comprising device. Devices such as a laptop, a mobile phone or a tablet may be portable devices. Hence, these devices may be used in a variety of locations at different times in the day. Hence, a well secured system ensures ease of use of the light generating system. Further, it may provide the advantage of protecting the device from damage, for example to prevent damage from a fall. Further, on a first use, the light generating system may be calibrated to provide light in a specific orientation based on how the user may choose to use the display comprising device. Hence, a well secured device may provide the advantage of maintaining the initial calibration of the device. Further, it may improve the convenience of usage without the requirement for frequent calibration of the device.

In embodiments of the arrangement, the display comprising device may be adjustable to a suitable viewing angle, thus, lighting module attached to the display comprising device, may not be aligned optimally to provide UV-B radiation to the user. This may inadvertently provide UV-B radiation, such as onto the eyes of the user. Hence, in embodiments of the arrangement, the lighting module may comprise one or more of a mirror, or a prism, or a combination thereof. The mirror or the prism may promote safe and reliable use of the device by directing the emitted light away from the eyes of the user. In further embodiments, the mirror or the prism may be used to direct light onto a particular region in front of the light source. Alternatively or additionally, in embodiments of the arrangement, the lighting module may be configured in an adjustable position relative to the display comprising device, such as via an adjustable hinge.

In yet another aspect, the invention may provide a method of providing UV radiation in a space, according to claim 13.

In embodiments, the invention may relate to using the light generating system, or the arrangement, to provide ultraviolet radiation to the user. According to the invention, the ultraviolet radiation have a wavelength in the 280-320 nm wavelength range. The radiation provided may have a maximum radiant exposure H_{a,max} selected from the range 30-900Jₐ/m², at a predetermined range dₚ selected from the range of up to 100 cm, such as up to 20 cm, or in embodiments selected from the values of 20 cm or 100 cm, or selected from the range of 20-100 cm. This may help ensure that the dosage provided to the user does not exceed the safe dosage limit. In embodiment, the maximum radiant exposure H_{a,max} may be selected from the range 30-300Jₐ/m².

In the art, radiant exposure H is also indicated as Hₑ. The radiant exposure may be defined as the radiant energy received by a surface per unit area, or equivalently irradiance of a surface integrated over time of irradiation. This is sometimes also called "radiant fluence".

The light generating system may be configured to switch off the light source when the maximum radiant exposure H_{a,max} is expected to be received by an object. On the basis of the proximity sensor signal and the power provided to the light source, the control system may determine the radiant exposure Hₐ which is expected to be received by an object (sensed by the proximity sensor). For instance, when a person is during a period exposed to UV-B radiation, the system may in embodiments determine the radiant exposure Hₐ which is received (by a sensed object), and when reaching the maximum radiant exposure H_{a,max} , the control system may switch off the light source. In other embodiments, the system may in embodiments determine the radiant exposure Hₐ which is (expected to be) received by a sensed object, and control the flux of the light source radiation such that the maximum radiant exposure H_{a,max} is not reached. For instance, the flux of the light source radiation may fade out over time, to prevent reaching the maximum radiant exposure H_{a,max} by the sensed object. The control system may also take into account movements of the person, leading to varying distances to the proximity sensor.

In embodiments, the method may further relate to regulating the intensity, duration, and operational mode of the lighting module based on a related proximity sensor signal.

In embodiments, the method may comprise providing visible light to the user. This may be advantageous in providing lighting without UV radiation.

The term "space" may for instance relate to a (part of) hospitality area, such as a restaurant, a hotel, a clinic, or a hospital, etc. The term "space" may also relate to (a part of) an office, a department store, a warehouse, a cinema, a church, a theatre, a library, etc. However, the term "space" may also relate to (a part of) a working space in a vehicle, such as a cabin of a truck, a cabin of an airplane, a cabin of a vessel (ship), a cabin of a car, a cabin of a crane, a cabin of an engineering vehicle like a tractor, etc. The term "space" may also relate to (a part of) a working space, such as an office, a (production) plant, a power plant (like a nuclear power plant, a gas power plant, a coal power plant, etc.), etc. For instance, the term "space" may also relate to a control room, a security room, etc. Especially, the term "space" may herein refer to an indoor space. In yet other embodiments, the term "space" may also relate to a toilet room or bathroom. In yet other embodiments, the term "space" may also relate to an elevator. In embodiments, the term "space" may also refer to a conference room, a school room, an indoor hallway, an indoor corridor, an indoor space in an elderly home, an indoor space in a nursing home, etc. In embodiments, the term "space" may refer to an indoor sport space, like a gym, a gymnastics hall, in indoor ball sport space, a ballet room, a swimming pool, a changing room, etc. In embodiments, the term "space" may refer to an (indoor) bar, an (indoor) disco, etc.

The terms "upstream" and "downstream" relate to an arrangement of items or features relative to the propagation of the light from a light generating means (here the especially the light source), wherein relative to a first position within a beam of light from the light generating means, a second position in the beam of light closer to the light generating means is "upstream", and a third position within the beam of light further away from the light generating means is "downstream".

The lighting device may be part of or may be applied in e.g. office lighting systems, household application systems, shop lighting systems, home lighting systems, accent lighting systems, spot lighting systems, theater lighting systems, fiber-optics application systems, projection systems, self-lit display systems, pixelated display systems, segmented display systems, warning sign systems, medical lighting application systems, indicator sign systems, decorative lighting systems, portable systems, automotive applications, (outdoor) road lighting systems, urban lighting systems, green house lighting systems, horticulture lighting, digital projection, or LCD backlighting.

The phrases "different light sources" or "a plurality of different light sources", and similar phrases, may in embodiments refer to a plurality of solid state light sources selected from at least two different bins. Likewise, the phrases "identical light sources" or "a plurality of same light sources", and similar phrases, may in embodiments refer to a plurality of solid state light sources selected from the same bin.

The term "white light" herein, is known to the person skilled in the art. It especially relates to light having a correlated color temperature (CCT) between about 1800 K and 20000 K, such as between 2000 and 20000 K, especially 2700-20000 K, for general lighting especially in the range of about 2700 K and 6500 K.

The terms "visible", "visible light" or "visible emission" and similar terms refer to light having one or more wavelengths in the range of about 380-780 nm.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Fig. 1 illustrates the light generating system 1000;
Fig. 2 illustrates the different positions of the slider element 420;
Fig. 3a-d illustrates the different ways of attaching the lighting module 100 to the display comprising device 1100; and
Fig. 4 illustrates the arrangement 2000.

The schematic drawings are not necessarily to scale.

### DETAILED DESCRIPTION OF THE EMBODIMENTS

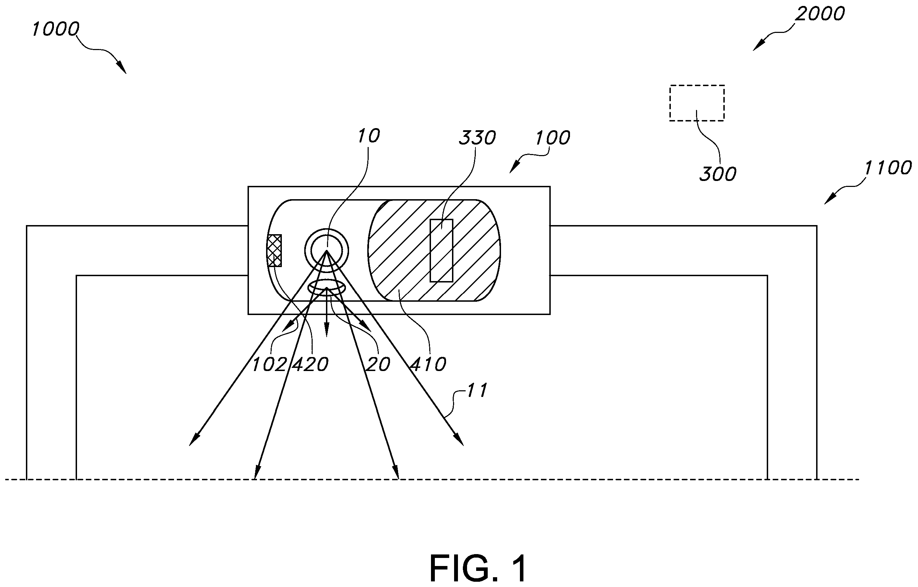

Fig. 1 schematically depicts an embodiment of the light generating system 1000, which may be functionally coupled to the display comprising device 1100 (of which schematically a part is shown). The light generating system 1000 further comprises the lighting module 100 which may be attached to the top of the screen of the display comprising device 1100. The light generating system 1000 further comprises the light source 10, which is configured to emit light source radiation 11 which comprises UV-B radiation. The light source 10 may especially be comprised by the lighting module.

Further, the light generating system may comprise a second light source 20 configured to provide visible light, indicated with reference 102. Hence, the light source 10 may optionally also be indicated as "first light source".

The light generating system 1000 further comprises a control system 300. In embodiments, the control may be part of the display comprising device 1100, such as a program or an application that can be executed on a computer.

In other embodiments, the control system 300 may be comprised by the lighting module.

The light generating system may further comprise a slider element 410. The slider element may be configured in two or more different positions. The slider element may slide in the downstream direction, first obstructing the field of view of the proximity sensor 330 and then obstructing the field of view of the light source 10. The light source is configured to provide light source radiation 11 which comprises UV light radiation 11. The proximity sensor 330 may be configured to detect the presence of entities in the field of view of the proximity sensor.

In Fig. 1, by way of example the slider element 410 blocks the proximity sensor 330 but not the light source 10.

Note that in Fig. 1 the optional second light source 20 may also be blocked by the slider element 410 when translated to the right. However, this aspect is only an example. The second light source 20 may, if available, also be positioned in such a way that it cannot be blocked by the slider element 410.

Fig. 1 also schematically depicts an arrangement 2000 comprising the light generating system 1000 and the display comprising device 1100.

During operation, the proximity sensor when detects the presence of an entity, generates a related proximity sensor signal. The control system 300 receives the proximity sensor signal. The control system 300 may further process the proximity sensor signal to infer positional information about the entities in the field of view of the proximity sensor 330. Based on this information, the control system 300 may estimate the amount of UV radiation 11 already provided to the user and may further estimate the amount of dosage that may be provided to ensure that a safe dosage of radiation is provided to the user. The control system 300 may regulate the light source 10 to configure the intensity of the radiation provided and the duration for which the radiation may be provided. The control system 300 may further regulate the intensity of UV radiation 11 provided based on the distance of the user from the proximity sensor 330. Upon reaching the safe dosage limit, the control system 300 may configure the light source to be in the off-mode. The control system 300 may further configure the light source 10 to be operated in the off-mode, if the related proximity sensor signal from the proximity sensor 330 indicates the presence of no user for a duration longer than a minute.

Hence, amongst others the invention provides a light generating system 1000 comprising a lighting module 100, wherein the lighting module 100 comprises a light source 10 configured to generate light source radiation 11 comprising UV radiation 11, a proximity sensor 330, and a control system 300. The UV radiation 11 comprises radiation having a wavelength in the 280-320 nm wavelength range. The proximity sensor 330 is configured to generate a proximity sensor signal in dependence of a presence of an object in a field of view of the proximity sensor 330. The control system 300 is be configured to control the light source 10 in dependence of the proximity sensor signal. The lighting module 100 is functionally attachable to a display comprising device 1100.

Further, the user may manually configure the light source 10 to be in the off-mode by moving the slider element 410 to a position where the control system 300 determines the light source 10 to be off and/or optionally to a position, where the slider element 410 may contact a tactile switch 420by which the light source 10 may be switched in the off mode.

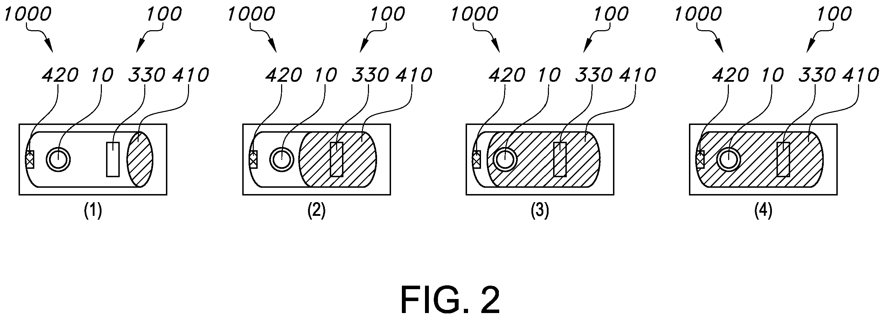

Fig. 2 schematically depicts an embodiment of the light generating system 1000, more especially the module 100, with the different positions of operating the slider element 410. Here, by way of example also tactile switches 420 are shown. Such tactile switches are not necessarily available, and may also be positioned at other positions. Further, a tactile switch 420 may be configured such, that upon contact with the tactile switch the light source 10 will be switched off, or that upon losing contact with the tactile switch the light source is switched off. Likewise, the tactile switch 420 may be configured such, that upon contact with the tactile switch the light source 10 will be switched on, or that upon losing contact with the tactile switch the light source is switched on.

In position (1), the slider element does not obstruct the proximity sensor 330, the light source 10, and or physically contact the optional tactile switch 420. The light source may be configured to provide UV radiation 11 in this position. In position (2), the slider element may be downstream of the proximity sensor 330, thus obstructing the field of view of the proximity sensor 330. This triggers the related proximity sensor signal indicating the presence of an object in the immediate vicinity of the proximity sensor 330. In this position, based on the proximity sensor signal, the control system 300 may configure the light source 10 to switch to the off-mode, hence providing no UV radiation 11.

Hence, the light generating system 1000 may further comprise a slider element 410. Especially, the slider element 410 may be slidable between a first position wherein the slider element 410 is neither configured downstream of the proximity sensor 330 nor of the light source 10 (see option 1 in Fig. 2), and a second position wherein the slider element 410 is configured downstream of the proximity sensor 330 (see option 2 in Fig. 2). Especially, in the second position the control system 300 (not depicted) is configured to keep the light source 10 in the off-mode in dependence of the proximity sensor signal.

In position (3), the slider element 410, is downstream of both the proximity sensor 330 and the light source 10. In this position, the field of view of the proximity sensor 330 remains obstructed and hence the light source 10 remains in the off-mode. Further, the slider element 410 may protect the user from accidental exposure to UV radiation 11 i.e. in the case that the light source 10 continues to provide UV radiation 10 erroneously. Position (3) provides the user the advantage of manually shielding the light source 10.

The three positions described herein, are a plurality of positions i.e. the positions are not discrete and there may be a range of positions for the slider element 410 to reside in. In other embodiments, these positions may also be discrete.

Hence, in embodiments the slider element 410 may be slidable between the first position (see option 1 in Fig. 2) and a third position (see option 3 in Fig. 2) wherein the slider element 410 is configured downstream of both the proximity sensor 330 and the light source 10.

Referring to option 4 in Fig. 2, position (4) is a discrete position wherein, the slider element 410 may physically contact the tactile switch 420. Physically contacting may refer to pressing the tactile switch 420 using the slider element 410. The tactile switch 420 may be placed downstream of the proximity sensor 330 and the light source 10. Position (4) may trigger the tactile sensor signal that may configure the light source 10 to switch to the off mode.

Hence, in embodiments the slider element 410 may be slidable in a fourth position downstream of the light source 10 and in one or more other positions. In embodiments, the light generating system may further comprise a tactile switch 420, wherein the slider element 410 may be configured to contact the tactile switch 420 when positioned in the fourth position. Especially, in (such) embodiments upon contacting the tactile switch 420, the control system 300 is configured to keep or switch the light source 10 in the off-mode.

However, in other embodiments the tactile switch 420 may also be placed in between the proximity sensor 330 and the light source 10. Other embodiments than depicted may be possible, wherein the tactile switch 420 is available or is not available.

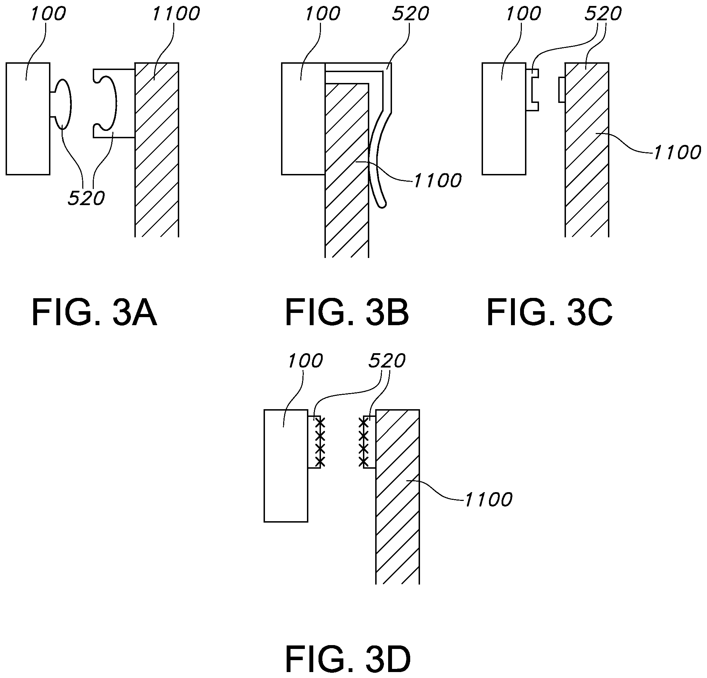

Fig. 3 schematically depicts some of the different embodiments illustrating how the lighting module 100 may be attached to the display comprising device 1100 by means of the attaching element 520. Fig. 3a depicts a snapper attaching element 520, where the ball part of the snapper may be attached to the back of lighting module 100. The socket part of the attaching element 520 may be a part of the display comprising device 1100. The ball part may snap into the socket part of the snapper attaching element 520. Fig. 3b depicts a clamp attaching element 520. The lighting module 100 may comprise a clamp attachment element 520 attached to the back of the lighting module 100. The lighting module 100 may be pressed down on top of an edge of the display comprising device 1100. The clamp attachment element 520 may be a spring that secures the lighting module 100 to the display comprising device 1100. Fig. 3c depicts a rail attachment element 520. The display comprising device 1100 may comprise a guide part and the lighting module 100 may comprise a rail part of the rail attachment element 520. This may allow the lighting module 100 to be secured along the guide part of the attachment element 520, hence securing the lighting module physically in place. Fig. 3d depicts a Velcro attachment element 520. The male part of the Velcro may be attached to the back of the lighting module 100 and the female part of the Velcro may be attached to the display comprising device 1100. This may allow the lighting module 100 to be hitched to the display comprising device 1100. These attachments may help secure the lighting module in place, while still providing some flexibility of movement. This may provide the advantage of being able to secure the lighting module 100 to the display comprising device 1100 in different orientations.

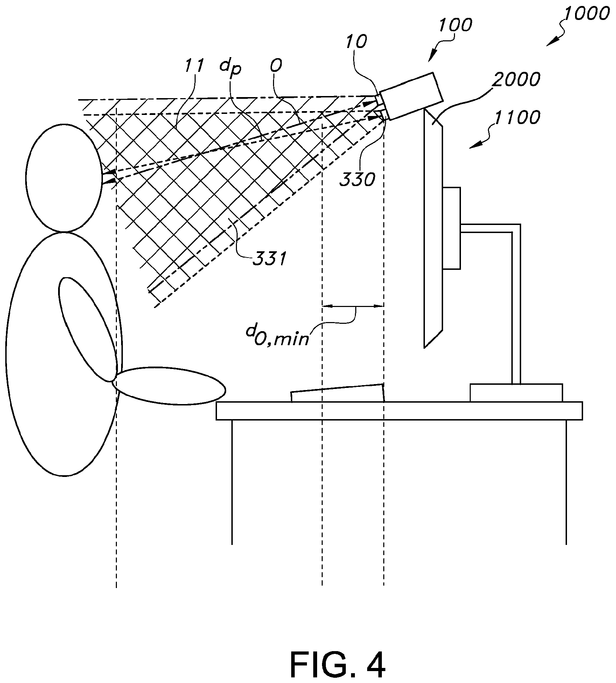

Fig. 4 schematically depicts an embodiment of the arrangement 2000 comprising the light generating system 1000 and the display comprising device 1100. The figure depicts a desk with a computer. The self-standing monitor in this embodiment may be the display comprising device 1100. The lighting module 100 is functionally coupled to the top of the monitor or the display comprising device 1100. The arrangement may be configured to provide UV light radiation 11 to the user.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" also includes embodiments wherein the term "comprises" means "consists of".

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

## Claims

1. A light generating system (1000) comprising (a) a lighting module (100), wherein the lighting module (100) comprises a light source (10) configured to generate light source radiation (11) comprising UV radiation (11), (b) a proximity sensor (330), and (c) a control system (300); wherein:
- the UV radiation (11) comprises radiation having a wavelength in the 280-320 nm wavelength range;
- the proximity sensor (330) is configured to generate a proximity sensor signal in dependence of a presence of an object in a field of view of the proximity sensor (330);
- the control system (300) is configured to control the light source (10) in dependence of the proximity sensor signal;
- the lighting module (100) is functionally attachable to a display comprising device (1100); and **characterised in that**:
(i) the proximity sensor (330) is configured to determine a distance (dₒ) between the object and the light source (10); the control system (300) is configured to determine a dosage Dₐ of UV radiation (11) received by the object on the basis of (i) a radiant flux of the UV radiation (11) over time and (b) the proximity sensor signal of the proximity sensor (330); and the control system (300) is configured to relate the dosage Dₐ of UV radiation (11) received by the object to a predetermined safe dosage limit Dₛ and on the basis thereon control the radiant flux of the UV radiation (11); and/or
(ii) the proximity sensor (330) is configured to estimate an average distance over time d_{o,a} between the object and the light source (10); the control system (300) is configured to estimate a dosage Dₐ of UV radiation (11) received and to be received by the object on the basis of (i) a radiant flux of the UV radiation (11) over time and (ii) the proximity sensor signal of the proximity sensor (330); and the control system (300) is configured to relate the dosage Dₐ of UV radiation (11) received by the object to a predetermined safe dosage limit Dₛ and on the basis thereon control the radiant flux of the UV radiation (11).

2. The light generating system (1000) according to claim 1, further comprising a slider element (410), wherein the slider element (410) is slidable between (i) a first position wherein the slider element (410) is neither configured downstream of the proximity sensor (330) nor of the light source (10) and (ii) a second position wherein the slider element (410) is configured downstream of the proximity sensor (330); wherein in the second position the control system (300) is configured to keep the light source (10) in the off-mode in dependence of the proximity sensor signal.

3. The light generating system (1000) according to claim 2, wherein the slider element (410) is slidable between (i) the first position and (iii) a third position wherein the slider element (410) is configured downstream of both the proximity sensor (330) and the light source (10).

4. The light generating system (1000) according to claim 3, wherein the slider element (410) is slidable in a fourth position downstream of the light source (10) and in one or more other positions, wherein the light generating system further comprises a tactile switch (420), wherein the slider element (410) is configured to contact the tactile switch (420) when positioned in the fourth position; wherein upon contacting the tactile switch (420), the control system (300) is configured to keep or switch the light source (10) in the off-mode.

5. The light generating system (1000) according to any one of the preceding claims, wherein the light source (10), at maximum power, is configured to provide the UV radiation (11) with a maximum radiant exposure E_{a,max} within a predetermined range (dₚ), wherein the maximum radiant exposure E_{a,max} is selected from the range of up to 30 mWa/m2, and wherein the predetermined range (dₚ) is selected from the range of up to 100 cm.

6. The light generating system (1000) according to any one of the preceding claims, wherein the proximity sensor (330) is selected from a group comprising a camera, a passive infrared sensor, an ultrasonic sensor, a microwave sensor, a time of flight sensor, and an audio sensor.

7. The light generating system (1000) according to any one of the preceding claims, wherein the control system (300) is configured to switch the light source (10) to the off-mode based on detecting the object within a predetermined minimum distance d_{o,min} from the light source (10) during a predetermined minimum duration t_{o,min}, wherein the predetermined minimum distance d_{o,min} is selected from the range of 0-20 cm, and wherein the predetermined minimum duration t_{o,min} is at least 0.1 seconds.

8. The light generating system (1000) according to any one of the preceding claims, wherein the lighting module (100) is configured to generate in a first operational mode the UV radiation (11) and in a second operational mode visible light (102), wherein the visible light (102) is white light having a CRI of at least 80.

9. The light generating system (1000) according to any one of the preceding claims, further comprising attaching elements (520) selected from the group of a rail, a magnet, a clamping element, a snapper element, and Velcro; wherein the attaching elements (520) are functionally coupled to the lighting module (100).

10. The light generating system (1000) according to any one of the preceding claims, wherein the lighting module (100) comprises the proximity sensor (330) and the control system (300).

11. An arrangement (2000) comprising the light generating system (1000) according to any one of the preceding claims and a display comprising device (1100), wherein the lighting module (100) is attached to the display comprising device (1100).

12. The arrangement (2000) according to claim 11, wherein the display comprising device (1100) is selected from the group of a self-standing monitor, a supported monitor or display, a laptop, and a tablet.

13. A method of providing UV radiation (11) in a space, wherein the method comprises using the light generating system (1000) according to any one of the preceding claims 1-10 or the arrangement according to any one of the preceding claims 11-12, and generating the UV radiation (11).

## Patentansprüche

1. Lichterzeugungssystem (1000), umfassend (a) ein Beleuchtungsmodul (100), wobei das Beleuchtungsmodul (100) eine Lichtquelle (10) umfasst, die konfiguriert ist, um Lichtquellenstrahlung (11) zu erzeugen, die UV-Strahlung (11) umfasst, (b) einen Näherungssensor (330) und (c) ein Steuersystem (300); wobei:
- die UV-Strahlung (11) Strahlung mit einer Wellenlänge im Wellenlängenbereich von 280-320 nm umfasst;
- der Näherungssensor (330) konfiguriert ist, um in Abhängigkeit von der Anwesenheit eines Objekts in einem Sichtfeld des Näherungssensors (330) ein Näherungssensorsignal zu erzeugen;
- das Steuersystem (300) konfiguriert ist, um die Lichtquelle (10) in Abhängigkeit von dem Näherungssensorsignal zu steuern;
- das Beleuchtungsmodul (100) funktionell an einer Vorrichtung mit Display (1100) befestigbar ist; und **dadurch gekennzeichnet, dass:**
(i) der Näherungssensor (330) konfiguriert ist, um eine Entfernung (dₒ) zwischen dem Objekt und der Lichtquelle (10) zu bestimmen; das Steuersystem (300) konfiguriert ist, um eine Dosierung Dₐ einer UV-Strahlung (11) zu bestimmen, die vom Objekt auf der Grundlage (i) eines Strahlungsflusses der UV-Strahlung (11) über die Zeit und (b) des Näherungssensorsignals des Näherungssensors (330) empfangenen wird; und das Steuersystem (300) konfiguriert ist, um die Dosierung Dₐ einer UV-Strahlung (11), die von dem Objekt empfangen wird, mit einer vorgegebenen sicheren Dosierungsgrenze Dₛ zu verknüpfen und auf dieser Grundlage den Strahlungsfluss der UV-Strahlung (11) zu steuern; und/oder
(ii) der Näherungssensor (330) konfiguriert ist, um eine durchschnittliche Entfernung über die Zeit d_{o,a} zwischen dem Objekt und der Lichtquelle (10) zu schätzen; das Steuersystem (300) konfiguriert ist, um eine Dosierung Dₐ einer UV-Strahlung (11) zu schätzen, die vom Objekt empfangen wurde und empfangen wird, auf der Grundlage (i) eines Strahlungsflusses der UV-Strahlung (11) über die Zeit und (ii) des Näherungssensorsignals des Näherungssensors (330); und das Steuersystem (300) konfiguriert ist, um die Dosierung Dₐ der UV-Strahlung (11), die vom Objekt empfangenen wurde, mit einer vorgegebenen sicheren Dosierungsgrenze Dₛ zu verknüpfen und auf dieser Grundlage den Strahlungsfluss der UV-Strahlung (11) zu steuern.

2. Lichterzeugungssystem (1000) gemäß Anspruch 1, das ferner ein Schieberelement (410) umfasst, wobei das Schieberelement (410) zwischen (i) einer ersten Position, in der das Schieberelement (410) weder dem Näherungssensor (330) noch der Lichtquelle (10) nachgeschaltet angeordnet ist, und (ii) einer zweiten Position, in der das Schieberelement (410) dem Näherungssensor (330) nachgeschaltet angeordnet ist, verschiebbar ist; wobei das Steuersystem (300) in der zweiten Position konfiguriert ist, um die Lichtquelle (10) in Abhängigkeit vom Näherungssensorsignal im Aus-Modus zu halten.

3. Lichterzeugungssystem (1000) gemäß Anspruch 2, wobei das Schieberelement (410) zwischen (i) der ersten Position und (iii) einer dritten Position verschiebbar ist, wobei das Schieberelement (410) sowohl dem Näherungssensor (330) als auch der Lichtquelle (10) nachgeschaltet angeordnet ist.

4. Lichterzeugungssystem (1000) gemäß Anspruch 3, wobei das Schieberelement (410) in eine vierte Position nachgeschaltet der Lichtquelle (10) und in eine oder mehrere andere Positionen verschiebbar ist, wobei das Lichterzeugungssystem ferner einen Tastschalter (420) umfasst, wobei das Schieberelement (410) konfiguriert ist, um den Tastschalter (420) zu berühren, wenn es in der vierten Position positioniert ist; wobei das Steuersystem (300) konfiguriert ist, um beim Berühren des Tastschalters (420) die Lichtquelle (10) im Aus-Modus zu halten oder in ihn zu schalten.

5. Lichterzeugungssystem (1000) gemäß einem der vorstehenden Ansprüche, wobei die Lichtquelle (10) bei maximaler Leistung konfiguriert ist, um die UV-Strahlung (11) mit einer maximalen Strahlungsintensität E_{a,max} innerhalb eines vorgegebenen Bereichs (dₚ) bereitzustellen, wobei die maximale Strahlungsintensität E_{a,max} aus dem Bereich von bis zu 30 mWa/m² ausgewählt wird, und wobei der vorgegebene Bereich (dₚ) aus dem Bereich bis 100 cm ausgewählt wird.

6. Lichterzeugungssystem (1000) gemäß einem der vorstehenden Ansprüche, wobei der Näherungssensor (330) aus einer Gruppe ausgewählt ist, die eine Kamera, einen passiven Infrarotsensor, einen Ultraschallsensor, einen Mikrowellensensor, einen Time-of-Flight-Sensor und einen Audiosensor umfasst.

7. Lichterzeugungssystem (1000) gemäß einem der vorstehenden Ansprüche, wobei das Steuersystem (300) konfiguriert ist, um die Lichtquelle (10) basierend auf der Erkennung des Objekts innerhalb einer vorgegebenen Mindestentfernung d_{o,min} von der Lichtquelle (10) während einer vorgegebenen Mindestdauer t_{o,min} in den Aus-Modus zu schalten, wobei die vorgegebene Mindestentfernung d_{o,min} aus dem Bereich von 0-20 cm ausgewählt wird, und wobei die vorgegebene Mindestdauer t_{o,min} mindestens 0,1 Sekunden beträgt.

8. Lichterzeugungssystem (1000) gemäß einem der vorstehenden Ansprüche, wobei das Beleuchtungsmodul (100) konfiguriert ist, um in einem ersten Betriebsmodus die UV-Strahlung (11) und in einem zweiten Betriebsmodus sichtbares Licht (102) zu erzeugen, wobei das sichtbare Licht (102) weißes Licht mit einem CRI von mindestens 80 ist.

9. Lichterzeugungssystem (1000) gemäß einem der vorstehenden Ansprüche, das ferner Befestigungselemente (520) umfasst, die aus der Gruppe ausgewählt sind, die aus einer Schiene, einem Magneten, einem Klemmelement, einem Schnappelement und einem Klettverschluss besteht; wobei die Befestigungselemente (520) funktional mit dem Beleuchtungsmodul (100) gekoppelt sind.

10. Lichterzeugungssystem (1000) nach einem der vorstehenden Ansprüche, wobei das Beleuchtungsmodul (100) einen Näherungssensor (330) und ein Steuersystem (300) umfasst.

11. Anordnung (2000), die das Lichterzeugungssystem (1000) gemäß einem der vorstehenden Ansprüche und eine Vorrichtung mit Display (1100) umfasst, wobei das Beleuchtungsmodul (100) an der Vorrichtung mit Display (1100) angebracht ist.

12. Anordnung (2000) gemäß Anspruch 11, wobei die Vorrichtung mit Display (1100) aus der Gruppe bestehend aus einem freistehenden Monitor, einem unterstützten Monitor oder Display, einem Laptop und einem Tablet ausgewählt ist.

13. Verfahren zum Bereitstellen von UV-Strahlung (11) in einem Raum, wobei das Verfahren das Verwenden des Lichterzeugungssystems (1000) gemäß einem der vorstehenden Ansprüche 1-10 oder der Anordnung gemäß einem der vorstehenden Ansprüche 11-12 und das Erzeugen der UV-Strahlung (11) umfasst.

## Revendications

1. Système générateur de lumière (1000) comprenant (a) un module d'éclairage (100), dans lequel le module d'éclairage (100) comprend une source de lumière (10) configurée pour générer un rayonnement de source de lumière (11) comprenant un rayonnement UV (11), (b) un capteur de proximité (330), et (c) un système de commande (300) ; dans lequel :
- le rayonnement UV (11) comprend un rayonnement ayant une longueur d'onde dans la plage de longueur d'onde de 280 à 320 nm ;
- le capteur de proximité (330) est configuré pour générer un signal de capteur de proximité en dépendance d'une présence d'un objet dans un champ de vision du capteur de proximité (330) ;
- le système de commande (300) est configuré pour commander la source de lumière (10) en dépendance du signal de capteur de proximité ;
- le module d'éclairage (100) peut être fixé fonctionnellement à un dispositif comprenant un affichage (1100) ; et **caractérisé en ce que :**
(i) le capteur de proximité (330) est configuré pour déterminer une distance (dₒ) entre l'objet et la source de lumière (10) ; le système de commande (300) est configuré pour déterminer une dose Dₐ de rayonnement UV (11) reçue par l'objet en fonction de (i) un flux rayonnant du rayonnement UV (11) au fil du temps et (b) le signal de capteur de proximité du capteur de proximité (330) ; et le système de commande (300) est configuré pour mettre en relation la dose Dₐ de rayonnement UV (11) reçue par l'objet avec une limite de dose sûre prédéterminée Dₛ et en fonction de celle-ci commander le flux rayonnant du rayonnement UV (11) ; et/ou
(ii) le capteur de proximité (330) est configuré pour estimer une distance moyenne au fil du temps d_{o,a} entre l'objet et la source de lumière (10) ; le système de commande (300) est configuré pour estimer une dose Dₐ de rayonnement UV (11) reçue et devant être reçue par l'objet en fonction de (i) un flux rayonnant du rayonnement UV (11) au fil du temps et (ii) le signal de capteur de proximité du capteur de proximité (330) ; et le système de commande (300) est configuré pour mettre en relation la dose Dₐ de rayonnement UV (11) reçue par l'objet avec une limite de dose sûre prédéterminée Dₛ et en fonction de celle-ci commander le flux rayonnant du rayonnement UV (11).

2. Système générateur de lumière (1000) selon la revendication 1, comprenant en outre un élément coulissant (410), dans lequel l'élément coulissant (410) peut coulisser entre (i) une première position dans lequel l'élément coulissant (410) n'est configuré en aval ni du capteur de proximité (330) ni de la source de lumière (10) et (ii) une deuxième position dans lequel l'élément coulissant (410) est configuré en aval du capteur de proximité (330) ; dans lequel dans la deuxième position le système de commande (300) est configuré pour garder la source de lumière (10) dans le mode arrêt en dépendance du signal de capteur de proximité.

3. Système générateur de lumière (1000) selon la revendication 2, dans lequel l'élément coulissant (410) peut coulisser entre (i) la première position et (iii) une troisième position dans lequel l'élément coulissant (410) est configuré en aval à la fois du capteur de proximité (330) et de la source de lumière (10).

4. Système générateur de lumière (1000) selon la revendication 3, dans lequel l'élément coulissant (410) peut coulisser dans une quatrième position en aval de la source de lumière (10) et dans une ou plusieurs autres positions, dans lequel le système générateur de lumière comprend en outre un commutateur tactile (420), dans lequel l'élément coulissant (410) est conçu pour venir en contact avec le commutateur tactile (420) lorsqu'il est positionné dans la quatrième position ; dans lequel lors d'un contact du commutateur tactile (420), le système de commande (300) est configuré pour garder ou commuter la source de lumière (10) dans le mode arrêt.

5. Système générateur de lumière (1000) selon l'une quelconque des revendications précédentes, dans lequel la source de lumière (10), à la puissance maximale, est configurée pour fournir le rayonnement UV (11) avec une exposition rayonnante maximale E_{a,max} au sein d'une plage prédéterminée (dₚ), dans lequel l'exposition rayonnante maximale E_{a,max} est choisie dans la plage allant jusqu'à 30 mWa/m2, et dans lequel la plage prédéterminée (dₚ) est choisie dans la plage allant jusqu'à 100 cm.

6. Système générateur de lumière (1000) selon l'une quelconque des revendications précédentes, dans lequel le capteur de proximité (330) est choisi parmi un groupe comprenant une caméra, un capteur infrarouge passif, un capteur à ultrasons, un capteur à micro-ondes, un capteur de temps de vol et un capteur audio.

7. Système générateur de lumière (1000) selon l'une quelconque des revendications précédentes, dans lequel le système de commande (300) est configuré pour commuter la source de lumière (10) au mode arrêt en fonction de la détection de l'objet au sein d'une distance minimale prédéterminée d_{o,min} par rapport à la source de lumière (10) pendant une durée minimale prédéterminée t_{o,min}, dans lequel la distance minimale prédéterminée d_{o,min} est choisie dans la plage de 0 à 20 cm, et dans lequel la durée minimale prédéterminée t_{o,min} vaut au moins 0,1 seconde.

8. Système générateur de lumière (1000) selon l'une quelconque des revendications précédentes, dans lequel le module d'éclairage (100) est configuré pour générer dans un premier mode fonctionnel le rayonnement UV (11) et dans un second mode fonctionnel de la lumière visible (102), dans lequel la lumière visible (102) est de la lumière blanche ayant un CRI d'au moins 80.

9. Système générateur de lumière (1000) selon l'une quelconque des revendications précédentes, comprenant en outre des éléments de fixation (520) choisis dans le groupe d'un rail, d'un aimant, d'un élément de serrage, d'un élément d'encliquetage et d'un Velcro ; dans lequel les éléments de fixation (520) sont accouplés fonctionnellement au module d'éclairage (100).

10. Système générateur de lumière (1000) selon l'une quelconque des revendications précédentes, dans lequel le module d'éclairage (100) comprend le capteur de proximité (330) et le système de commande (300).

11. Agencement (2000) comprenant le système générateur de lumière (1000) selon l'une quelconque des revendications précédentes et un dispositif comprenant un affichage (1100), dans lequel le module d'éclairage (100) est fixé au dispositif comprenant un affichage (1100).

12. Agencement (2000) selon la revendication 11, dans lequel le dispositif comprenant un affichage (1100) est choisi dans le groupe d'un moniteur autonome, d'un moniteur ou écran supporté, d'un ordinateur portable et d'une tablette.

13. Procédé de fourniture de rayonnement UV (11) dans un espace, dans lequel le procédé comprend l'utilisation du système générateur de lumière (1000) selon l'une quelconque des revendications 1 à 10 précédentes ou l'agencement selon l'une quelconque des revendications 11 à 12 précédentes, et la génération du rayonnement UV (11).
